# EUROPEAN PATENT SPECIFICATION

(11) **EP 4 389 179 B1**
(45) Date of publication and mention of the grant of the patent: **18.03.2026**
(21) Application number: 21953705.7
(22) Date of filing: 18.08.2021
(51) Int. Cl.: A61M 11/00, A61K 8/97, A61K 8/67, A61K 8/65, A61K 8/63, A61Q 5/00, A61Q 7/00, A45D 19/00, A45D 19/02, A61K 8/9789, A61M 35/00, A61Q 19/00, A61K 8/04

(54) **COSMETIC TREATMENT METHOD AND FORMULATION FOR SCALP AND HAIR, AND PRODUCT THEREOF**
KOSMETISCHES BEHANDLUNGSVERFAHREN UND FORMULIERUNG FÜR KOPFHAUT UND HAAR SOWIE PRODUKT DARAUS
PROCÉDÉ DE TRAITEMENT COSMÉTIQUE ET FORMULATION POUR CUIR CHEVELU ET CHEVEUX, ET PRODUIT ASSOCIÉ

(43) Date of publication of application: 26.06.2024
(73) Proprietor: Hou, Mau-Sheng, Taipei City, Taiwan 111 (TW); Wang, Pei-Ying, Taipei City, Taiwan 111 (TW)
(72) Inventor: Hou, Mau-Sheng, Taipei City, Taiwan 111 (TW); Wang, Pei-Ying, Taipei City, Taiwan 111 (TW)
(74) Representative: Charrier Rapp & Liebau
(86) International application number: PCT/CN2021/113151
(87) International publication number: WO 2023/019454

(56) References cited:
- EP-A2- 2 090 377
- WO-A1-2017/054806
- WO-A1-2017/054806
- CN-A- 101 618 375
- CN-A- 106 491 465
- CN-U- 212 067 374
- CN-U- 213 076 476
- CN-U- 213 820 189
- DE-A1- 102015 206 789
- DE-A1- 102015 206 789
- JP-A- 2006 006 831
- US-A1- 2019 350 334

## Description

### Technical Field

The present invention pertains to a beauty treatment method, formulation, and product for the scalp and hair, specifically referring to a water-containing cosmetic formulation and product that can be atomized using ultrasonic atomization technology, along with its application for beauty treatment on the scalp and hair.

### Technical Background

When applying cosmetics or medicinal products to the human skin, it is crucial for maximizing the product effectiveness by optimizing transdermal absorption. However, the physiological function for human skin as a natural barrier restricted the exchange between under-skin tissue and the environment. The natural barrier function of skin was contributed primarily by interconnected dead corneocytes. In addition, naturally produced sweat and sebum make it challenging for cosmetic or medicinal ingredients to penetrate and reach primary target tissues such as the dermis, epidermis, or subcutaneous tissues. Hence, within the realm of cosmetics, enhancing skin absorption has become a pivotal research topic.

EP 2 090 377 A2 relates to a specific spraying device, in the form of a spray, comprising a liquid cosmetic composition for use as a make-up product comprising itself at least one water-soluble dye. The liquid cosmetic composition has a viscosity of less than 250 mPa·s at 25 ° C and is sprayed in the form of droplets by means of the spraying device. The spraying device is comprising a container containing the liquid cosmetic composition and is equipped with a piezoelectric spraying mechanism.

The hair and skin of human scalp differ significantly from other areas of the body. Hair is the only continuously growing hair on the human body, and its structure consists primarily of the medulla, cortex, and cuticle layers from the inside out. The cortex, comprising 70% to 80% of the hair volume, is the main structure of the hair and is composed of protein fibers that readily bond with water. The moisture content in hair significantly influences its strength and appearance. The cuticle layer, also known as the hair scales, grows from the follicle end towards the tip, overlapping like bamboo shoot skin with slightly raised outer edges, forming gaps of about 3 micrometers under normal conditions. The surface of these scales contains natural lipids that protect the inner hair and act as a barrier against external damage. Factors such as washing, dyeing, and heat treatments reduce the moisture content in the cortex, affecting the overall health and appearance of the hair. When the gaps between the scales widen, hair tends to become frizzy. Among the hair care products, aside from altering its color, curl pattern, or shape, replenishing moisture to the hair and smoothing the hair scales are often key objectives.

DE 10 2015 206 789A1 relates to a device for applying a hair cosmetic preparation, in the form of finely atomised or atomized droplets, to the hair and / or the scalp. In order to achieve as complete and uniform wetting of the hair sections to be treated with hair cosmetic preparation, the device comprises at least one chamber for holding the hair cosmetic preparation, an ultrasonic atomizer, which on the hair cosmetic Preparation acts to nebulize this, a fluidic conveying device for the nebulized, hair cosmetic preparation, an energy source for electrical supply u. a. the ultrasonic atomizer and the conveying device and at least one dispensing opening for dispensing the hair cosmetic preparation.

The scalp boasts the highest density of hair follicles on the human body, with the number of hair reaching up to a hundred thousand due to genetic, age, and gender variations among individuals. These follicles come equipped with sebaceous glands, and when combined with numerous sweat gland pores, they create a sebum film on the scalp under normal physiological conditions. The continuous secretion of sebum and sweat provides a growth environment for bacteria or fungi, making the scalp the area with the highest microbial count on the body's skin. When the balance of oils and moisture is disrupted due to physiological, pathological, or environmental changes, it often leads to fluctuations in microbial populations. The secretions from these microbes further impact scalp health and hair growth. The follicles on the scalp differ significantly from those on other parts of the body. Scalp follicles typically grow 1 to 4 hairs simultaneously, and their openings form a funnel shape. In a healthy state, an adequate amount of sebum secretion and its outward movement can keep microbes from entering lower part of the follicle. On the contrary, excessive or insufficient sebum secretion can lead to inflammation either at the opening region or penetrate deep into follicles. Therefore, properly supplementing hair moisture or increasing the hydration of scalp cells can enhance the health and aesthetic appeal of the scalp and hair.

Hair follicles, besides their normal physiological functions, also serve as a pathway for the absorption of cosmetics or medicinal products through the scalp. With funnel-shaped openings approximately 60 to 70 micrometers in size, hair follicles offer larger passageways compared to sweat glands or the skin's stratum corneum. Located deeper within the dermis, hair bulbs are close to the subcutaneous microvasculature. Therefore, by miniaturizing cosmetic or medicinal ingredients, it is possible to conduct these active components through the gaps around the follicle openings and the stratum corneum, facilitating the delivery of active ingredients to deeper layers of the skin, thereby enhancing absorption.

When applying cosmetics to the scalp or hair, the influence of gravity often leads to draining and loss of the product. Consequently, many scalp and hair care products aim to extend their adhering time by adding increasing product viscosity with high molecular weight polymers. However, the scalp surface is not a smooth plane; it is a complex three-dimensional structure formed by pores, hair, and layers of dead corneocytes. High viscosity rendered high surface tension of the formulation, leading to the formation of gaps when the formula comes into contact with the skin structure. This does not actually increase the contact surface area. High-viscosity cosmetics are also challenging for post-application cleansing to remove the redundant product especially around the initial application sites. Other formulation methods to enhance ingredient penetration may introduce compositions that physically or chemically alter either hair protein structure or scalp corneocyte integrity. However, these methods often necessitate disrupting normal cell structures, leading to a different type of physiological damage.

Another research area to enhance the absorption of cosmetic or medicinal ingredients is through the technique of liquid atomization.

Liquid atomization has found widespread applications across various fields, including industries like manufacturing, agriculture, medicine, and more. The purposes of utilizing liquid atomization differ across these domains, encompassing objectives such as increasing total liquid surface area to enhance combustion rates, improving efficiency in surface coating processes, enhancing precision in spray printing, boosting plant fertilizer absorption, and expediting drug delivery. The use of atomization technology in the cosmetics field aligns with objectives in the medical field. Both aim to reduce the unit volume of liquid, disperse the total volume, and increase the surface area to promote enhanced skin contact and absorption.

According to the energy transmission method, atomization technology can be classified into two main types: mechanical atomization and ultrasonic atomization. Mechanical atomization encompasses methods like pressure atomization, two-fluid atomization, and rotary disc spraying. Despite its widespread use across various domains, mechanical atomization has drawbacks such as higher energy consumption and difficulties in controlling the size and velocity of the aerosol particles.

Using pressurized atomization is a known method to spray cosmetics onto the scalp and hair. Pressurized atomization has the advantage of relatively shorter application time. However, its spraying speed becomes both an advantage and a drawback. When the mist droplets hit the surface, the high inbound speed causes a rebound effect, leading to a significant portion of the components scattering into the air, reducing the time they stay on the scalp or hair surface.

The ultrasonic atomization technique involves electrically controlling piezoelectric elements to vibrate at frequencies reaching millions of times per second, generating fine standing waves on a liquid film to atomize the liquid by separating it from the wavefront. By modulating factors like oscillation frequency, power, and the physical-chemical properties of the liquid, this technique can produce mist droplets with an average diameter of 10 micrometers or even submicron levels. Moreover, its size distribution tends to be more concentrated compared to mechanical atomization. Therefore, for industries highly dependent on droplet size, such as drug delivery, ultrasonic atomization proves more suitable than mechanical atomization.

Although there have been studies proposing the application of cosmetics or medicinal components to the scalp using ultrasonic atomization, the mist generated by ultrasonic atomization tends to have lower flight speeds, making it prone to suspension in the air before eventually settling due to gravity. Consequently, the deposition location of these droplets is influenced by the contours of the contact surface. In practical implementation on the scalp, there is a need to navigate through the physical barriers presented by hair shaft, ensuring the droplets settle on the surface of the scalp or preferably reach the openings of the hair follicles. Only then can the advantages of ultrasonic atomization such as smaller and more concentrated droplet size be fully leveraged to enhance the absorption of cosmetics or medicinal components.

The majority of hair follicle openings are not perpendicular to the skin but rather align with the direction of hair flow, forming angles ranging from 0 to 90 degrees with the skin surface. Therefore, these funnel openings have a specific orientation. Additionally, smearing and massaging has been shown to enhance the scalp absorption and delivery of liquid formulas to the deeper layers of the skin.

Currently, there is not a beauty method that involves applying cosmetic or medicinal ingredients onto the scalp through ultrasonic atomization, followed by scalp massage to enhance absorption and effectiveness.

### Summary of the Invention

This invention provides a beauty treatment method for the scalp and hair according to claim 1 and the use of a water-containing cosmetic formula in the beauty treatment method, according to claim 11, wherein the water-containing cosmetic formula is applicable to the scalp and hair and is capable of being atomized using ultrasonic technology. It aims to enhance scalp cell hydration and replenish hair moisture, thus improving the overall health and aesthetic appearance of the scalp and hair.

According to one embodiment of this invention, the water-containing cosmetic formula possesses a viscosity ranging from 0.5 mPa·S to 1000 mPa·S and a water content between 1 and 99 wt.%.

According to one embodiment of this invention, the water-containing cosmetic formula comprises at least one component selected from the following: 5 wt.% German chamomile, 1 wt.% Vitamin B5, 3 wt.% hydrolyzed keratin, and 1 wt.% sodium glycyrrhizinate. The present invention provides a beauty treatment method for the scalp and hair, comprising the following steps: loading the water-containing cosmetic formula into an ultrasonic atomizer; using the ultrasonic atomizer to atomize the water-containing cosmetic formula; lifting the hair to expose the scalp underneath in the direction against hair stream; positioning the ultrasonic oscillation piece of the ultrasonic atomizer close to the scalp; spraying the atomized water-containing cosmetic formula onto the scalp in the direction against hair stream; and massaging the scalp in the direction against hair stream.

According to one embodiment of the present invention, the beauty treatment method for the scalp and hair involves spraying the atomized water-containing cosmetic formula onto the scalp in the direction against hair stream for a duration ranging from 5 to 20 minutes.

According to one embodiment of the present invention, the beauty treatment method for the scalp and hair involves massaging the scalp in the direction against hair stream for a duration ranging from 5 to 20 minutes.

According to one embodiment of the present invention, the beauty treatment method for the scalp and hair involves maintaining a minimum linear distance between the ultrasonic oscillation piece of the ultrasonic atomizer and the surface of the scalp, ranging from 0.1 centimeters to 5 centimeters.

According to one embodiment of the present invention, the beauty treatment method for the scalp and hair involves setting the vibration frequency of the ultrasonic oscillation piece of the ultrasonic atomizer between twenty kilohertz and four million hertz.

According to one embodiment of the present invention, the beauty treatment method for the scalp and hair involves utilizing the ultrasonic atomizer to atomize the water-containing cosmetic formula into mist droplets with an average diameter ranging from 10 nanometers to 100 micrometers.

On another front, the present invention provides a cosmetic product suitable for the scalp and hair, comprising the water-containing cosmetic formula and an ultrasonic atomizer suitable for atomizing the water-containing cosmetic formula into mist droplets with an average diameter ranging from 10 nanometers to 100 micrometers.

According to one embodiment of the present invention, the ultrasonic atomizer contain at least one piezoelectric element, controlled electrically to oscillate at a frequency between twenty kilohertz and four million hertz.

### Brief Description of the Drawings

Figure 1 illustrates the steps of the beauty treatment method for the scalp and hair in the present invention.
Figure 2 illustrates the direction of hair stream and the direction against hair stream.
Figure 3 illustrates the comparison of scalp moisture levels when spraying in the direction of hair stream versus the direction against hair stream.
Figure 4 illustrates the comparison of scalp moisture levels between not massaging and massaging in the direction against hair stream.
Figure 5 illustrates the comparison of scalp moisture levels after using water-containing cosmetic formulas with varying water contents.

Key Symbol Description: 101-106 - Process steps in the beauty treatment method for scalp and hair.

### Embodiments

The following sections will provide a detailed description of the embodiments of the present invention in conjunction with the accompanying figures.

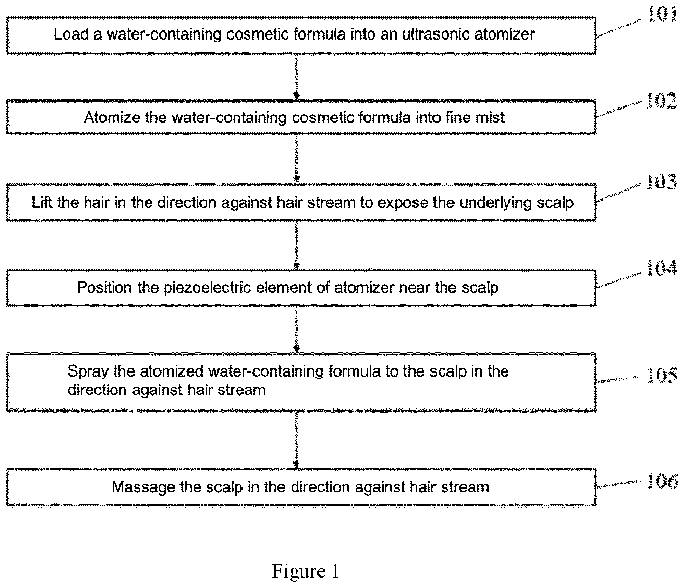

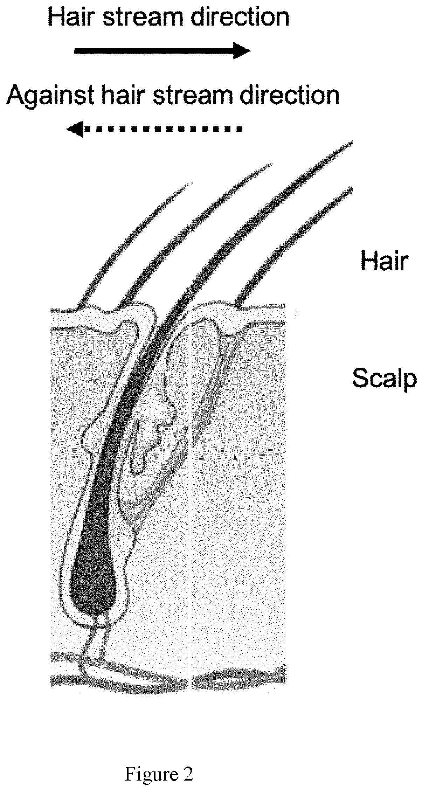

Please refer to Figure 1, illustrating the process steps of a beauty treatment method for the scalp and hair according to an embodiment of the present invention. Firstly, at step 101, a water-containing cosmetic formula is obtained and loaded into an ultrasonic atomizer. The water-containing cosmetic formula has a water content ranging from 1 to 99 weight percent and a viscosity between 0.5 mPa·S and 1000 Pa·S. This cosmetic formula may comprise one or more main cosmetic ingredients to provide various effects on the hair and scalp, including but not limited to moisturizing, antibacterial, oil control, deodorizing, anti-dandruff, cleansing, anti-inflammatory, soothing, repairing, hair growth, strengthening, and reducing hair loss. For instance, the water-containing cosmetic formula may further include at least one component chosen from the following: 5wt.% German chamomile, 1wt.% Vitamin B5, 3wt.% hydrolyzed keratin, and 1wt.% sodium glycyrrhizinate. The ultrasonic atomizer utilized in the present invention may contain at least one piezoelectric element. Furthermore, the ultrasonic atomizer of the present invention can be a handheld device, enhancing user convenience. Next, at step 102, the piezoelectric element of the ultrasonic atomizer is electrically controlled to oscillate at a frequency between twenty kilohertz and four million hertz, facilitating the atomization of the water-containing cosmetic formula through the ultrasonic atomizer, resulting in mist droplets with an average diameter ranging from 10 nanometers to 100 micrometers emitted from the ultrasonic atomizer. At step 103, as shown in Figure 2, the hair is lifted in the direction against hair stream to expose the underlying scalp. Subsequently, at step 104, the ultrasonic atomizer is brought close to the exposed scalp, positioning its piezoelectric element near the scalp, for instance, the shortest linear distance between the piezoelectric element and the scalp being between 0.1 centimeters and 5 centimeters. Following this, at step 105, the atomized water-containing cosmetic formula is sprayed onto the exposed scalp in the direction against hair stream, with an optimal spray duration of 5 to 20 minutes, preferably 5 minutes. Lastly, at step 106, the scalp is massaged in the direction against hair stream for an optimal duration of 5 to 20 minutes, preferably 5 minutes.

The beauty treatment method for the scalp and hair according to this invention is not restricted to the sequence depicted in Figure 1. Steps 102 and 103 can be implemented before step 104.

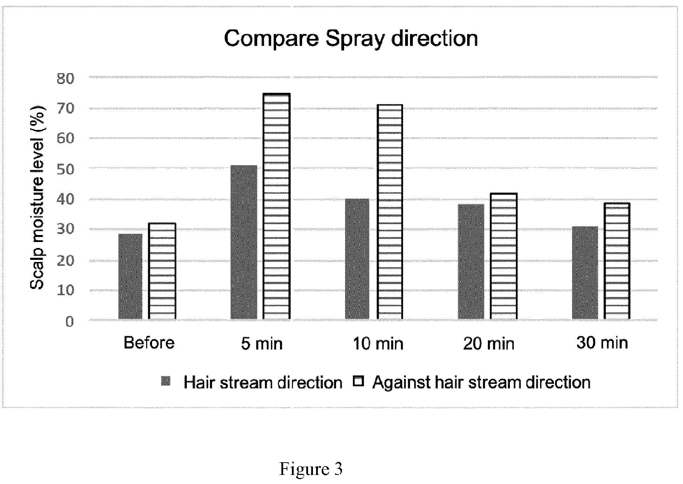

The characteristic of the water-containing cosmetic formula of this invention enables its ultrasonic atomization technology to form fine mist droplets with an average diameter ranging from 10 nanometers to 100 micrometers, thus facilitating skin/scalp absorption. Figure 3 illustrates the comparison of scalp moisture levels after applying the ultrasonic mist sprayer of this invention, spraying the atomized water-containing cosmetic formula in both the direction of hair stream and the opposite direction (i.e., against the hair stream). It can be observed from Figure 3 that after five minutes of spraying the water-containing cosmetic formula atomized by the ultrasonic sprayer of this invention against the hair stream, the moisture level of the scalp exceeds 75%. However, when sprayed in the direction of hair stream, the moisture level of the scalp after five minutes is approximately 48%.

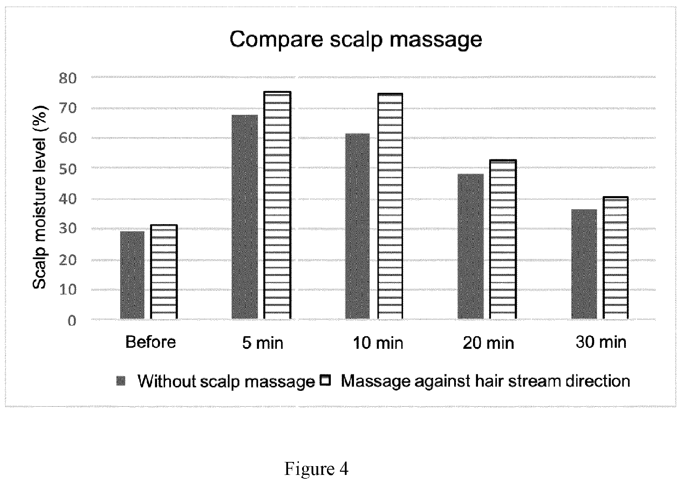

Figure 4 illustrates the comparison of scalp moisture levels after applying the atomized water-containing cosmetic formula of this invention to the scalp, with and without scalp massage. From Figure 4, it is evident that massaging the scalp for five, ten, or twenty minutes after applying the atomized water-containing cosmetic formula of this invention leads to an approximate 10% increase in scalp moisture compared to not massaging the scalp.

According to the test results from Figures 3 and 4, the water-containing cosmetic formula provided by this invention can be atomized into fine mist droplets that facilitate skin absorption with ultrasonic atomization technology. Furthermore, the beauty treatment method for scalp and hair introduced by this invention effectively enhances the moisture retention of both the scalp and hair.

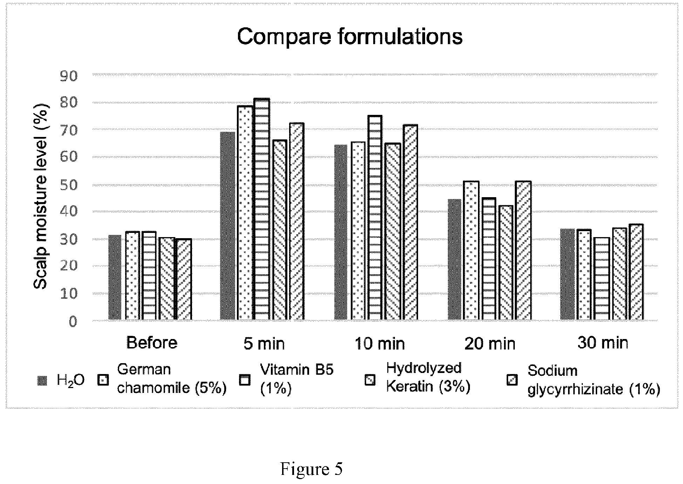

Figure 5 illustrates the comparison of the moisturizing effects on the scalp after spraying different water-containing cosmetic formulas, with varying water content levels, atomized through the ultrasonic atomization technology provided by this invention. From Figure 5, it is evident that spraying these different water-containing cosmetic formulas onto the scalp for five, ten, or twenty minutes results in a 10% to 50% increase in scalp moisture compared to before application. Particularly, the best effect is observed when the ultrasonically atomized water-based cosmetic formula is sprayed onto the scalp for five minutes, showing a remarkable 40% to 50% increase in scalp moisture compared to before application.

On the other hand, this invention also provides a cosmetic product suitable for the scalp and hair. It involves packaging the water-containing cosmetic formula provided by this invention into an ultrasonic atomizer capable of atomizing said formula into fine mist droplets averaging between 10 nanometers to 100 micrometers in diameter. The cosmetic product of this invention may encompass the packaging of the water-containing cosmetic formula, designed to be suitable for atomizing it into fine mist droplets averaging between 10 nanometers to 100 micrometers in diameter, within a handheld ultrasonic atomizer.

Based on the embodiment mentioned above, it demonstrates that the water-containing cosmetic formula provided by this invention can be atomized using ultrasound technology and applied to the scalp and hair. Utilizing the present method for scalp and hair beauty treatment indeed enhances scalp cell hydration and replenishes hair moisture, thereby improving the health and aesthetics of the scalp and hair.

## Claims

1. A beauty treatment method for the scalp and hair, comprising:
loading a water-containing cosmetic formula into an ultrasonic atomizer;
using the ultrasonic atomizer to atomize the water-containing cosmetic formula;
lifting the hair to expose the scalp underneath in a direction against hair stream;
positioning one or more ultrasonic oscillation piece of the ultrasonic atomizer close to the scalp;
spraying the atomized water-containing cosmetic formula onto the scalp in the direction against hair stream; and
massaging the scalp in the direction against hair stream.

2. The method of claim 1, wherein the step of spraying the atomized water-containing cosmetic formula onto the scalp in the direction against hair stream is for a duration ranging from 5 to 20 minutes.

3. The method of claim 1, wherein the step of massaging the scalp in the direction against hair stream is for a duration ranging from 5 to 20 minutes.

4. The method of claim 1, wherein maintaining a minimum linear distance between the one or more ultrasonic oscillation piece of the ultrasonic atomizer and the surface of the scalp ranging from 0.1 centimeters to 5 centimeters.

5. The method of claim 1, wherein the water-containing cosmetic formula has a viscosity between 0.5 mPa·S and 1000 mPa·S.

6. The method of claim 1, wherein the water-containing cosmetic formula has a water content ranging from 1 to 99 weight percent.

7. The method of claim 1, wherein the water-containing cosmetic formula includes at least one component chosen from the following: 5wt.% German chamomile, 1wt.% Vitamin B5, 3wt.% hydrolyzed keratin, and 1wt.% sodium glycyrrhizinate.

8. The method of claim 1, wherein the one or more ultrasonic oscillation piece of the ultrasonic atomizer oscillates at a frequency between twenty kilohertz and four million hertz.

9. The method of claim 1, wherein the one or more ultrasonic oscillation piece contains at least one piezoelectric element.

10. The method of claim 1, wherein the step of using the ultrasonic atomizer to atomize the water-containing cosmetic formula atomizes the water-containing cosmetic formula into mist droplets with an average diameter ranging from 10 nanometers to 100 micrometers.

11. Use of a water-containing cosmetic formula in the beauty treatment method for the scalp and hair according to claim 1, wherein the water-containing cosmetic formula for the scalp and hair has a viscosity between 0.5 mPa·S and 1000 mPa·S and a water content ranging from 1 to 99 weight percent.

12. Use according toclaim11, wherein the water-containing cosmetic formula is further comprising at least one component chosen from the following: 5wt.% German chamomile, 1wt.% Vitamin B5, 3wt.% hydrolyzed keratin, and 1wt.% sodium glycyrrhizinate.

13. Use according to claim 11, wherein the water-containing cosmetic formula is atomized by an ultrasonic atomizer into fine mist droplets averaging between 10 nanometers to 100 micrometers in diameter.

14. Use according to claim 13, wherein the ultrasonic atomizer contains at least one piezoelectric element which oscillates at a frequency between twenty kilohertz and four million hertz.

## Patentansprüche

1. Ein Schönheitsbehandlungsverfahren für die Kopfhaut und das Haar, umfassend:
Einfüllen einer wasserhaltigen kosmetischen Formulierung in einen Ultraschallzerstäuber;
Verwenden des Ultraschallzerstäubers zum Zerstäuben der wasserhaltigen kosmetischen Formulierung;
Anheben der Haare, um die darunter liegende Kopfhaut entgegen der Haarwuchsrichtung freizulegen;
Positionieren eines oder mehrerer Ultraschall-Oszillationselemente des Ultraschallzerstäubers nahe der Kopfhaut;
Sprühen der zerstäubten wasserhaltigen kosmetischen Formulierung auf die Kopfhaut entgegen der Haarwuchsrichtung; und
Massieren der Kopfhaut entgegen der Haarwuchsrichtung.

2. Verfahren nach Anspruch 1, wobei der Schritt des Sprühens der zerstäubten wasserhaltigen kosmetischen Formulierung auf die Kopfhaut entgegen der Haarwuchsrichtung eine Dauer von 5 bis 20 Minuten hat.

3. Verfahren nach Anspruch 1, wobei der Schritt des Massierens der Kopfhaut entgegen der Haarwuchsrichtung eine Dauer von 5 bis 20 Minuten hat.

4. Verfahren nach Anspruch 1, wobei ein Mindestabstand zwischen dem einen oder den mehreren Ultraschallschwingungselementen des Ultraschallzerstäubers und der Oberfläche der Kopfhaut von 0,1 Zentimetern bis 5 Zentimetern eingehalten wird.

5. Verfahren nach Anspruch 1, wobei die wasserhaltige kosmetische Formulierung eine Viskosität zwischen 0,5 mPa·S und 1000 mPa·S aufweist.

6. Verfahren nach Anspruch 1, wobei die wasserhaltige kosmetische Formulierung einen Wassergehalt im Bereich von 1 bis 99 Gewichtsprozent aufweist.

7. Verfahren nach Anspruch 1, wobei die wasserhaltige kosmetische Formulierung mindestens einen Bestandteil enthält, der aus den folgenden ausgewählt ist: 5 Gew.-% deutsche Kamille, 1 Gew.-% Vitamin B5, 3 Gew.-% hydrolysiertes Keratin und 1 Gew.-% Natriumglycyrrhizinat.

8. Verfahren nach Anspruch 1, wobei das eine oder die mehreren Ultraschallschwingungselemente des Ultraschallzerstäubers mit einer Frequenz zwischen 20 Kilohertz und 4 Millionen Hertz schwingen.

9. Verfahren nach Anspruch 1, wobei das eine oder die mehreren Ultraschallschwingungsstücke mindestens ein piezoelektrisches Element enthalten.

10. Verfahren nach Anspruch 1, wobei der Schritt der Verwendung des Ultraschallzerstäubers zum Zerstäuben der wasserhaltigen kosmetischen Formulierung die wasserhaltige kosmetische Formulierung in feinste Nebeltröpfchen mit einem durchschnittlichen Durchmesser im Bereich von 10 Nanometern bis 100 Mikrometern zerstäubt.

11. Verwendung einer wasserhaltigen kosmetischen Formulierung in dem Verfahren zur Schönheitsbehandlung für die Kopfhaut und das Haar gemäß Anspruch 1, wobei die wasserhaltige kosmetische Formulierung für die Kopfhaut und das Haar eine Viskosität zwischen 0,5 mPa·S und 1000 mPa·S und einen Wassergehalt im Bereich von 1 bis 99 Gewichtsprozent aufweist.

12. Verwendung gemäß Anspruch 11, wobei die wasserhaltige kosmetische Formulierung ferner mindestens einen Bestandteil umfasst, der aus den folgenden ausgewählt ist: 5 Gew.-% deutsche Kamille, 1 Gew.-% Vitamin B5, 3 Gew.-% hydrolysiertes Keratin und 1 Gew.-% Natriumglycyrrhizinat.

13. Verwendung gemäß Anspruch 11, wobei die wasserhaltige kosmetische Formulierung durch einen Ultraschallzerstäuber in feine Nebeltröpfchen mit einem durchschnittlichen Durchmesser zwischen 10 Nanometern und 100 Mikrometern zerstäubt wird.

14. Verwendung gemäß Anspruch 13, wobei der Ultraschallzerstäuber mindestens ein piezoelektrisches Element enthält, das mit einer Frequenz zwischen 20 Kilohertz und 4 Millionen Hertz schwingt.

## Revendications

1. Procédé de traitement de beauté pour le cuir chevelu et les cheveux, comprenant :
le chargement d'une formule cosmétique contenant de l'eau dans un atomiseur ultrasonique ;
l'utilisation de l'atomiseur ultrasonique pour atomiser la formule cosmétique contenant de l'eau ;
le soulèvement des cheveux pour exposer le cuir chevelu en dessous dans une direction contre le courant de cheveux ;
le positionnement d'une ou plusieurs pièces d'oscillation ultrasonique de l'atomiseur ultrasonique près du cuir chevelu ;
la pulvérisation de la formule cosmétique atomisée contenant de l'eau sur le cuir chevelu dans la direction opposée au courant capillaire ; et
le massage du cuir chevelu dans le sens opposé au flux de cheveux.

2. Procédé selon la revendication 1, dans lequel l'étape de pulvérisation de la formule cosmétique atomisée contenant de l'eau sur le cuir chevelu dans la direction opposée au courant de cheveux est d'une durée comprise entre 5 et 20 minutes.

3. Procédé selon la revendication 1, dans lequel l'étape de massage du cuir chevelu dans la direction opposée au flux de cheveux est d'une durée comprise entre 5 et 20 minutes.

4. Procédé selon la revendication 1, dans lequel le maintien d'une distance linéaire minimale entre la ou les pièces d'oscillation ultrasoniques de l'atomiseur ultrasonique et la surface du cuir chevelu allant de 0,1 centimètre à 5 centimètres.

5. Procédé selon la revendication 1, dans lequel la formule cosmétique contenant de l'eau a une viscosité comprise entre 0,5 mPa•s et 1 000 mPa•S.

6. Procédé selon la revendication 1, dans lequel la formule cosmétique contenant de l'eau a une teneur en eau allant de 1 à 99 pour cent en poids.

7. Procédé selon la revendication 1, dans lequel la formule cosmétique contenant de l'eau comporte au moins un composant choisi parmi ce qui suit : 5 % en poids de camomille allemande, 1 % en poids de vitamine B5, 3 % en poids de kératine hydrolysée et 1 % en poids de glycyrrhizinate de sodium.

8. Procédé selon la revendication 1, dans lequel la ou les pièces d'oscillation ultrasoniques de l'atomiseur ultrasonique oscillent à une fréquence comprise entre vingt kilohertz et quatre millions de hertz.

9. Procédé selon la revendication 1, dans lequel la ou les pièces d'oscillation ultrasoniques contiennent au moins un élément piézoélectrique.

10. Procédé selon la revendication 1, dans lequel l'étape d'utilisation de l'atomiseur ultrasonique pour atomiser la formule cosmétique contenant de l'eau atomise la formule cosmétique contenant de l'eau en gouttelettes de brouillard d'un diamètre moyen allant de 10 nanomètres à 100 micromètres.

11. Utilisation d'une formule cosmétique contenant de l'eau dans le procédé de traitement de beauté du cuir chevelu et des cheveux selon la revendication 1, dans laquelle la formule cosmétique contenant de l'eau pour le cuir chevelu et les cheveux a une viscosité comprise entre 0,5 mPa•S et 1000 mPa•S et une teneur en eau allant de 1 à 99 pour cent en poids.

12. Utilisation selon la revendication 11, dans laquelle la formule cosmétique contenant de l'eau comprend en outre au moins un composant choisi parmi ce qui suit : 5 % en poids de camomille allemande, 1 % en poids de vitamine B5, 3 % en poids de kératine hydrolysée et 1 % en poids de glycyrrhizinate de sodium.

13. Utilisation selon la revendication 11, dans laquelle la formule cosmétique contenant de l'eau est atomisée par un atomiseur ultrasonique en fines gouttelettes de brouillard d'un diamètre moyen compris entre 10 nanomètres et 100 micromètres.

14. Utilisation selon la revendication 13, dans laquelle l'atomiseur ultrasonique contient au moins un élément piézoélectrique qui oscille à une fréquence comprise entre vingt kilohertz et quatre millions de hertz.
